# EUROPÄISCHE PATENTSCHRIFT

(11) **EP 1 192 912 B1**
(45) Veröffentlichungstag und Bekanntmachung des Hinweises auf die Patenterteilung: **12.01.2005**
(21) Anmeldenummer: 01116887.9
(22) Anmeldetag: 11.07.2001
(51) Int. Cl.: A61B 17/88, B25B 23/10

(54) **Hülsenförmige Vorrichtung zum Halten von Schrauben beim Eindrehen in einen Gegenstand, z. B. in einen Knochen, mit Hilfe eines Schraubendrehers**
Sheath-like device for holding screws during screwing into a substrate, e.g a bone, by means of a screwdriver
Dispositif en forme de manchon pour tenir de vis pendant le visage dans un substrat, par example un os, par un tournevis

(30) Priorität: 12.08.2000 DE 20013905 U
(43) Veröffentlichungstag der Anmeldung: 03.04.2002
(73) Patentinhaber: Stryker Trauma GmbH, 24232 Schönkirchen (DE)
(72) Erfinder: Simon, Bernd, 24106 Kiel (DE); Prien, Ole, 24145 Kiel (DE)
(74) Vertreter: Patentanwälte Hauck, Graalfs, Wehnert, Döring, Siemons, Schildberg

(56) Entgegenhaltungen:
- DE-U- 8 531 467
- US-A- 5 605 080

## Beschreibung

Die Erfindung bezieht sich auf eine hülsenförmige Vorrichtung zum Halten von Schrauben beim Eindrehen in einen Gegenstand, z. B. in einen Knochen, mit Hilfe eines Schraubendrehers nach dem Anspruch 1.

Derartige Vorrichtungen sind allgemein bekannt. Sie erleichtern z. B. dem Chirurgen das Eindrehen von Knochenschrauben oder ähnlichen Schrauben unter schlechten Handhabungs- und Sichtbedingungen. Der Spreizabschnitt dient zur Aufnahme der Schraube, insbesondere des Schraubenkopfes, und ermöglicht mithin die Aufnahme einer Schraube an einem vom Gegenstand entfernten Ort. Der Schaft des Schraubendrehers kann sich hierbei bereits innerhalb der Fanghülse befinden und seine Klinge oder sein entsprechendes Angriffsende mit der Schraube in Eingriff gebracht sein. Die auf der Fanghülse verschiebbare Sicherungshülse dient dazu, den Spreizabschnitt zu verriegeln, damit während des Einschraubvorgangs die Schraube in der Fanghülse gefangen ist. Zu diesem Zweck ist die Sicherungshülse zwischen zwei Positionen auf der Fanghülse gleitend bewegbar. In der vorderen Position nimmt sie ihre Sperrstellung ein, und in der hinteren Position ermöglicht sie das Aufspreizen des Spreizabschnitts zur Aufnahme einer Schraube bzw. zum Entfernen von der eingedrehten Schraube.

Solch eine Vorrichtung ist beispiels weise in DE-U-8 531 467 offenbart.

Die Sicherungshülse wird üblicherweise durch eine Feder in die Sperrstellung vorgespannt. Beim Einsetzen einer derartigen Vorrichtung besteht jedoch die Gefahr, daß z. B. durch umgebende Weichteile die Sicherungshülse beim Einführen der Vorrichtung an die Stelle, an der eine Schraube eingedreht werden soll, gegen die Feder zurückgeschoben wird, wodurch die Sicherungsfunktion der Sicherungshülse nicht mehr gewährleistet ist.

Der Erfindung liegt daher die Aufgabe zugrunde, eine hülsenförmige Vorrichtung zum Halten von Schrauben beim Eindrehen in einen Gegenstand mit Hilfe eines Schraubendrehers zu schaffen, bei der die Sicherungshülse unabhängig von angreifenden äußeren Kräften ihre Sperrposition beibehält.

Diese Aufgabe wird durch die Merkmale des Anspruchs 1 gelöst.

Erfindungsgemäß ist die Sicherungshülse auf der Fanghülse verriegelt, und zwar z.B. mit Hilfe mindestens einer Kugel, die in eine Ringnut der Fanghülse eingreift und von einem inneren Abschnitt des zweiten Griffabschnitts an einer Bewegung nach außen gehindert ist. Wird der zweite Griffabschnitt relativ zur Sicherungshülse bzw. Fanghülse bewegt, findet eine Relativbewegung zwischen den Hülsen zunächst nicht statt, d. h. die Sicherungshülse kann mithin nicht aus der Sperrposition in die Freigabeposition bewegt werden. Erst wenn der zweite Griffabschnitt eine Relativstellung erreicht hat, in der die Kugel in eine Ausnehmung des zweiten Griffabschnitts ausweichen kann, kann die zuvor komprimierte Feder zwischen Grifiabschnitt und einer Schulter der Sicherungshülse die Sicherungshülse wieder gegenüber dem zweiten Griffabschnitt zu einem Anschlag hin bewegen in eine Freigabestellung. Der zweite Griffabschnitt hat sich während seiner Bewegung von der Sicherungshülse zunächst fortbewegt unter Kompression der ersten Feder. Nach der Freigabe der Verriegelung kann die Sicherungshülse unter Entspannung der ersten Feder nachrutschen, so daß nunmehr der Spreizabschnitt eine Schraube aufnehmen bzw. freigeben kann. Der Schaft des Schraubendrehers, der durch die Fanghülse hindurch geführt ist, wird mit Hilfe einer weiteren Verriegelungskugel festgelegt, welche in eine Ringnut des Schaftes eingreift. Ein Abschnitt des zweiten Griffabschnitts verhindert auch hier normalerweise ein Herausbewegen der Verriegelungskugel. Sollen die beiden Teile voneinander gelöst werden, muß der zweite Griffabschnitt um eine vorgegebene Strecke relativ zur Fanghülse bewegt werden gegen die Kraft der weiteren Feder, bis die genannte Verriegelungskugel in eine weitere Ausnehmung des zweiten Griffabschnitts eintritt. Dabei ist die Strecke, welche hierbei zurückgelegt werden muß, größer als die Strecke, welche eine Relativbewegung zwischen zweitem Griffabschnitt und Sicherungshülse notwendig ist, damit diese in die Freigabestellung bewegt werden kann.

Aus den obigen Ausführungen ergibt sich, daß in der Sperrstellung die Sicherungshülse an einer axialen Bewegung in die Freigabestellung gehindert ist, mithin durch von außen angreifende Kräfte nicht verstellt werden kann. Solche Kräfte treten z.B. auf, wenn die Anordnung durch Gewebe geführt wird, bis die aufgenommene Schraube an Ort und Stelle eingeschraubt wird, z.B. in einen Knochen.

Nach einer Ausgestaltung der Erfindung ist die erste Feder vorzugsweise schwächer als die zweite Feder. Nach einer weiteren Ausgestaltung der Erfindung weist der zweite Griffabschnitt einen Hülsenabschnitt und einen Handhabungsabschnitt auf, die in Wirkverbindung miteinander stehen. Zum Beispiel kann der Hülsenabschnitt auf einen axialen Bund des Handhabungsabschnitts aufgeschraubt sein.

Nach einer anderen Ausgestaltung der Erfindung ist die zweite innere Schulter des zweiten Griffabschnitts und die radiale Ausnehmung von einem Ring gebildet, der innerhalb des zweiten Griffabschnitts angeordnet ist. Der Ring kann z. B. in den hülsenförmigen Abschnitt des zweiten Griffabschnitts eingeschraubt sein. Die beschriebenen Ausnehmungen, in welche die Verriegelungskugeln ausweichen können, sind vorzugsweise ringförmig im Inneren des zweiten Griffabschnitts geformt.

Die Erfindung wird nachfolgend anhand eines in einer Zeichnung dargestellten Ausführungsbeispiels näher erläutert.

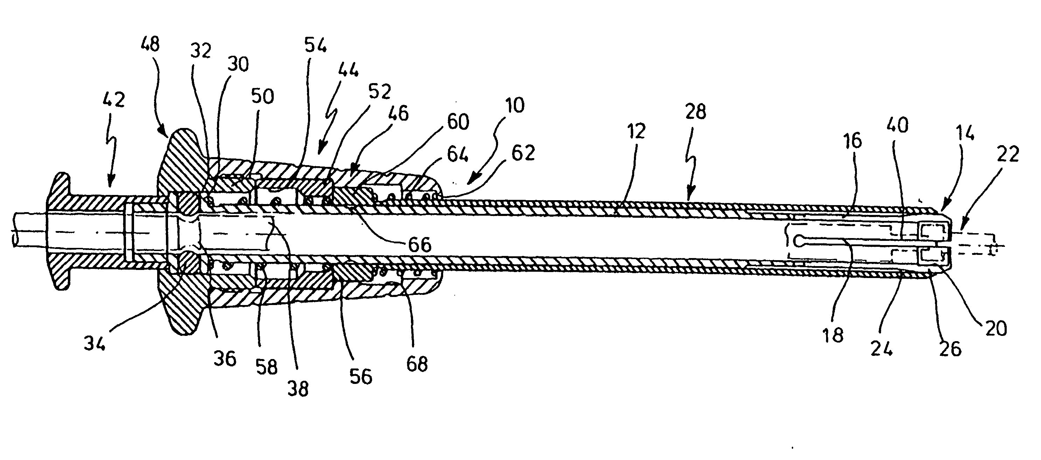

Die einzige Zeichnung zeigt einen Schnitt durch die Vorrichtung nach der Erfindung mit andeutungsweise gezeigtem Schraubendreher.

Die in der Figur dargestellte Haltevorrichtung 10 weist eine innere Fanghülse 12 auf mit einem Spreizabschnitt 14 am in der Figur rechten Ende, der aus einzelnen federnden Segmenten 16 besteht, die in Umfangsrichtung durch Schlitze 18, die achsparallel verlaufen, getrennt sind. Die Segmente 16 bilden am rechten oder freien Ende innen eine Ausnehmung 20 zur Aufnahme des Kopfes einer gestrichelt gezeichneten Schraube 22. Eine solche Fanghülse ist an sich allgemein bekannt. Die Segmente 16 weisen an der Außenseite eine Rampenfläche 24 auf, die nach rechts in einen verdickten Abschnitt 26 übergeht.

Auf der Fanghülse 12 gleitet eine Sicherungshülse 28, die in der Figur in ihrer Sperrstellung gezeigt ist. Sie überdeckt die verdickten Abschnitte 26 und verhindert, daß die Segmente 16 sich radial nach außen spreizen. Eine aufgenommene Schraube 22 wird mithin an einem Herausfallen oder Lösen gehindert.

Die Fanghülse 12 hat am linken Ende einen radialen Bund 30, der in gegenüberliegenden Bohrungen 32 Verriegelungskugeln 34 hält. Wie in der Zeichnung zu erkennen, wirken die Verriegelungskugeln mit einer Ringnut 36 eines Schaftes 38 eines nicht weiter dargestellten Schraubendrehers zusammen. Der Schaft 38 ist durch die gesamte Fanghülse 12 hindurch geführt, und eine Klinge 40 oder dergleichen wirkt mit dem Kopf der Schraube 22 zusammen, um diese in einen nicht gezeigten Gegenstand, z. B. einen Knochen, einzudrehen.

Auf dem in der Figur linken Ende der Fanghülse 12 sitzt ein hülsenförmiger erster Griffabschnitt 42 mit einer Bohrung, die koaxial zur Bohrung der Fanghülse 12 liegt. Ein zweiter Griffabschnitt 44 umgibt die Hülsen 12, 28 am linken Ende und den rechten Abschnitt des ersten Griffabschnitts 42. Der Griffabschnitt 44 weist einen außen konischen Hülsenabschnitt 46 auf und einen Handhabungsabschnitt 48, wobei der linke Teil des Hülsenabschnitts 46 auf einen axialen Bund 50 des Handhabungsabschnitts 48 aufgeschraubt ist. Zwischen dem Bund 50 und einer inneren Schulter des zweiten Griffabschnitts 44 sitzt ein Ring 52 im Hülsenabschnitt 46. Der Ring hat eine radiale ringförmige Ausnehmung 54, und zwischen einer inneren radialen Schulter 56 des Rings 52 und dem radialen Bund 30 der Fanghülse 12 ist eine Schraubenfeder 58 angeordnet.

Zwischen einem radialen Bund 60 und einer inneren radialen Schulter 62 des Hülsenabschnitts 46 ist eine Feder 64 angeordnet. Sie ist ebenfalls eine Schraubenfeder. Der radiale Bund 60 nimmt mindestens eine nicht gezeigte Verriegelungskugel auf, die in einer entsprechenden Bohrung radial beweglich ist und in der in der Figur gezeigten Position der Teile radial nach außen nicht entweichen kann. Sie wirkt mit einer äußeren Ringnut 66 der Fanghülse 12 zusammen. Der Hülsenabschnitt 46 weist eine innere Ringausnehmung 68 auf, in welche die genannte Verriegelungskugel radial entweichen kann, wenn sich der Ringbund 60 innerhalb der Ausnehmung 68 befindet.

Bei der Beschreibung der Funktion der gezeigten Vorrichtung wird von der Darstellung in der Figur ausgegangen, in welcher der Spreizabschnitt 14 den Kopf einer Schraube aufgenommen hat und die Sicherungshülse 28 den Spreizabschnitt sperrt. Mit Hilfe des aufgenommenen Schraubendrehers kann nun eine Schraube in einen Gegenstand eingedreht werden. Die Verriegelung der beiden Hülsen 12, 28 gegeneinander verhindert, daß durch von außen einwirkende Kräfte die Sicherungshülse 28 nach hinten geschoben und der Spreizabschnitt aufgespreizt werden kann. Soll nun die Vorrichtung von der Schraube 22 gelöst werden, wird der Handhabungsabschnitt 48 in Richtung auf den ersten Griffabschnitt 42 zu gezogen unter Kompression der Federn 58, 64, ohne daß die Sicherungshülse 28 ihre Position relativ zur Fanghülse 12 aufgibt. Dadurch entfernt sich der Ring 52 von dem radialen Bund 60, und zwar so lange, bis der Bund 60 sich in der Ausnehmung 68 befindet und die Verriegelungskugel radial entweichen kann. Ist dies der Fall, drückt die Feder 64 die Sicherungshülse 28 wieder in Richtung Ring 52 bis in Anschlag an diesen. Dadurch nimmt die Sicherungshülse 28 ihre Freigabestellung ein, und durch Aufspreizen des Spreizabschnitts 14 kann die Vorrichtung vom Kopf der Schraube 22 entfernt werden. Für gewöhnlich wird der Griff 44 noch weiter zurückgezogen, bis die Kugeln 34 in die Ausnehmung 54 rutschen und dadurch ein Verschieben der gesamten Anordnung auf dem Schaft 38 ermöglichen gegen einen nicht gezeigten Absatz, gegen den der erste Griffabschnitt ausschlägt, um eine definierte Position einzunehmen.

## Patentansprüche

1. Hülsenförmige Vorrichtung zum Halten von Schrauben beim Eindrehen in einen Gegenstand, z. B. einen Knochen, mit Hilfe eines Schraubendrehers, mit
- einer Fanghülse (12), die am freien Ende einen radial spreizbaren Spreizabschnitt (14) zur Aufnahme der Schraube (22) und am anderen Ende mindestens eine radiale Bohrung zur beweglichen Aufnahme einer ersten Verriegelungskugel (34) aufweist, die mit einer Ringnut (36) im Schaft (38) eines Schraubendrehers zusammenwirkt,
- einer auf der Fanghülse (12) gleitend angeordneten Sicherungshülse (28), die in einer gegenüber der Fanghülse (12) vorderen Stellung eine Spreizung des Spreizabschnitts (14) sperrt und in einer gegenüber der Fanghülse (12) zurückgezogenen Stellung den Spreizabschnitt (14) freigibt,
- einem mit der Fanghülse (12) verbundenen ersten Griffabschnitt (42)
- einem hülsenförmigen zweiten Griffabschnitt (44), der Fanghülse (12) und Sicherungshülse (28) umgibt und gegenüber dem ersten Griffabschnitt (42) axial beweglich ist und die erste Verriegelungskugel (34) in Eingriff mit einer Ringnut (36) des Schraubendreherschaftes (38) hält,
- einer ersten Feder (64) zwischen einer inneren Schulter (62) an dem dem Spreizabschnitt (14) zugewandten Ende des zweiten Griffabschnitts (44) und einer radialen Schulter der Sicherungshülse (28),
- mindestens einer zwischen Sicherungshülse (28) und Fanghülse (12) wirkenden Verriegelung, welche die Sicherungshülse (28) in der Sperrstellung relativ zur Fanghülse (12) axial sichert und von dem zweiten Griffabschnitt (44) betätigt wird, wenn er eine vorgegebene Strecke relativ zu den Hülsen (12, 28) bewegt wird.

2. Vorrichtung nach Anspruch 1, mit
- einer zweiten Verriegelungskugel, die in einer radialen Bohrung eines Bundes (60) der Sicherungshülse (28) beweglich gelagert ist, mit einer Ringnut (66) der Fanghülse (12) zusammenwirkt und von dem zweiten Griffabschnitt (44) in Eingriff mit der zweiten Ringnut (66) gehalten wird,
- einer zweiten Feder (58) zwischen einer weiteren radialen Schulter des zweiten Griffabschnitts (44) und einer radialen Schulter der Fanghülse (12),
- einer inneren radialen Ausnehmung (68) in dem zweiten Griffabschnitt (44), in welche die zweite Verriegelungskugel eintreten kann, wenn der zweite Griffabschnitt (44) relativ zur Sicherungshülse (28) eine vorgegebene erste Strecke bewegt wird unter Kompression der ersten und zweiten Feder (62, 58), wodurch die erste Feder (64) die Sicherungshülse (28) in die Freigabestellung drückt, und
- einer zweiten inneren radialen Ausnehmung (54) im zweiten Griffabschnitt (44), in welche die erste Verriegelungskugel (34) eintritt, wenn der zweite Griffabschnitt (44) relativ zur Fanghülse (12) bzw. zum ersten Griffabschnitt (42) eine vorgegebene zweite Strecke bewegt wird unter Kompression der zweiten Feder (58), wobei die zweite Strecke länger als die erste ist.

3. Vorrichtung nach Anspruch 1 oder 2, **dadurch gekennzeichnet, daß** die erste Feder (64) schwächer als die zweite Feder (58) ist.

4. Vorrichtung nach einem der Ansprüche 1 bis 3, **dadurch gekennzeichnet, daß** der zweite Griffabschnitt (44) einen Hülsenabschnitt (46) und einen Handhabungsabschnitt (48) aufweist, die in Wirkverbindung stehen.

5. Vorrichtung nach einem der Ansprüche 1 bis 4 **dadurch gekennzeichnet, daß** die zweite innere Schulter des zweiten Griffabschnitts (44) und die radiale Ausnehmung (54) von einem Ring (52) gebildet sind, der innerhalb des zweiten Griffabschnitts (44) angeordnet ist.

## Claims

1. A sleeve-shaped device to retain screws while these are turned into an object such as a bone by means of a screw-driver, comprising:
- a catch sleeve (12) the free end of which has a radially expandable portion (14) for receiving the screw (22) and the other end of which has at least one radial bore for movably receiving a first locking ball (34) which interacts with an annular groove (36) in the shank (38) of a screw-driver,
- a safety sleeve (28) slidingly disposed on the catch sleeve (12), which blocks an expansion of the expandable portion (14) in a position which is at front with respect to the catch sleeve (12) and releases the expandable portion (14) in a position which is retracted with respect to the catch sleeve (12),
- a first handle portion (42) connected to the catch sleeve (12),
- a sleeve-shaped, second handle portion (44) which encircles the catch sleeve (12) and the safety sleeve (28) and is axially movable with respect to the first handle portion (42) and holds the first locking ball (34) in engagement with an annular groove (36) of the screw-driver shank (38),
- a first spring (64) between an inner shoulder (62) at the end of the second handle portion (44) facing the expandable portion (14) and a radial shoulder of the safety sleeve (28),
- at least an interlocking mechanism acting between the safety sleeve (28) and the catch sleeve (12), which axially secures the safety sleeve (28) in a blocking position relative to the catch sleeve (12) and is actuated by the second handle portion (44) if it is moved a predetermined distance relative to the sleeves (12, 28).

2. The device according to claim 1, comprising:
- a second locking ball which is movably mounted in a radial bore of a collar (60) of the safety sleeve (28), interacts with an annular groove (66) of the catch sleeve (12), and is held by the second handle portion (44) in engagement with the second annular groove (66),
- a second spring (58) between another radial shoulder of the second handle portion (44) and a radial shoulder of the catch sleeve (12),
- an inner radial recess (68) in the second handle portion (44), which can be entered by the second locking ball if the second handle portion (44) is moved a predetermined first distance relative to the safety sleeve (28) while the first and second springs (62, 58) are compressed, which causes the first spring (64) to urge the safety sleeve (28) into the releasing position, and
- a second inner radial recess (54) in the second handle portion (44) which the second locking ball (34) enters if the second handle portion (44) is moved a predetermined second distance relative to the safety sleeve (12) and the first handle portion (42) when the second spring (58) is compressed, with the second distance being longer than the first one.

3. The device according to claim 1 or 2, **characterized in that** the first spring (64) is weaker than the second spring (58).

4. The device according to any one of claims 1 to 3, **characterized in that** the second handle portion (44) has a sleeve portion (46) and a handling portion (48) which are in an operative connection..

5. The device according to any one of claims 1 to 4, **characterized in that** the second inner shoulder of the second handle portion (44) and the radial recess (54) are defined by a ring (52) which is disposed within the second handle portion (44).

## Revendications

1. Dispositif en forme de douille destinée à maintenir des vis pendant le vissage au moyen d'un tournevis dans un objet, tel qu'un os, comportant
- une douille de retenue (12), dont l'extrémité libre comporte une zone d'expansion (14) expansible dans le sens radial et destinée à recevoir la vis (22) et dont l'autre extrémité comporte au moins un alésage radial destiné à recevoir de manière mobile une première bille de verrouillage (34), qui coopère avec une rainure circulaire (36) dans la tige (38) d'un tournevis,
- une douille de sûreté (28), qui est montée coulissante sur la douille de retenue (12) et qui, dans une position avant par rapport à la douille de retenue (12), empêche une expansion de la zone d'expansion (14) et, dans une position en retrait par rapport à la douille de retenue (12), libère la zone d'expansion (14),
- un premier manche (42) relié à la douille de retenue (12),
- un deuxième manche (44) en forme de douille, qui enveloppe la douille de retenue (12) et la douille de sûreté (28) et est mobile axialement par rapport au premier manche (42) et retient la première bille de verrouillage (34) en prise dans la rainure circulaire (36) de la tige (38) du tournevis,
- un premier ressort (64) entre un épaulement intérieur (62) au niveau de l'extrémité du deuxième manche (44), orientée vers la zone d'expansion (14), et un épaulement radial de la douille de sûreté (28),
- au moins un verrouillage opérant entre la douille de sûreté (28) et la douille de retenue (12), par lequel la douille de sûreté (28) est bloquée en position de verrouillage axialement par rapport à la douille de retenue (12) et qui est actionné par le deuxième manche (44) lorsque celui-ci est déplacé sur une distance prédéfinie par rapport aux douilles (12, 28).

2. Dispositif selon la revendication 1, comportant
- une deuxième bille de verrouillage, qui est logée mobile dans un alésage radial d'une bride (60) de la douille de sûreté (28), coopère avec une rainure circulaire (66) de la douille de retenue (12) et est maintenue par le deuxième manche (44) en prise dans la deuxième rainure circulaire (66),
- un deuxième ressort (58) entre un autre épaulement radial du deuxième manche (44) et un épaulement radial de la douille de retenue (12),
- un évidement radial (68) intérieur dans le deuxième manche (44), dans lequel peut s'engager la deuxième bille de verrouillage, lorsque le deuxième manche (44) est déplacé par rapport à la douille de sûreté (28) sur une première distance prédéfinie, moyennant la compression du premier et du deuxième ressort (62, 58), à la suite de quoi le premier ressort (64) pousse la douille de blocage (28) dans la position de libération, et
- un deuxième évidement (54) radial intérieur dans le deuxième manche (44), dans lequel s'engage la première bille de verrouillage (34), lorsque le deuxième manche (44) est déplacé par rapport à la douille de retenue (12) et au premier manche (42) sur une deuxième distance prédéfinie, moyennant la compression du deuxième ressort (58), la deuxième distance étant plus longue que la première.

3. Dispositif selon la revendication 1 ou 2, **caractérisé en ce que** le premier ressort (64) est plus faible que le deuxième ressort (58).

4. Dispositif selon l'une quelconque des revendications 1 à 3, **caractérisé en ce que** le deuxième manche (44) comporte un tronçon formant une douille (46) et un tronçon de maniement (48), lesquels sont en liaison active.

5. Dispositif selon l'une quelconque des revendications 1 à 4, **caractérisé en ce que** le deuxième épaulement intérieur du deuxième manche (44) et l'évidement radial (54) sont formés par une bague (52), qui est agencée à l'intérieur du deuxième manche (44).
